# EUROPÄISCHE PATENTSCHRIFT

(11) **EP 3 386 422 B1**
(45) Veröffentlichungstag und Bekanntmachung des Hinweises auf die Patenterteilung: **18.09.2019**
(21) Anmeldenummer: 16805830.3
(22) Anmeldetag: 05.12.2016
(51) Int. Cl.: A61B 50/30, A61L 2/26

(54) **MEDIZINISCHE UMLAUFSPERRVORRICHTUNG, MEDIZINISCHES INSTRUMENT UND MEDIZINISCHER STERILISIERBEHÄLTER**
MEDICAL LOCKING DEVICE, MEDICAL INSTRUMENT AND MEDICAL STERILIZATION CONTAINER
DISPOSITIF DE BLOCAGE EN ROTATION À USAGE MEDICAL, INSTRUMENT À USAGE MÉDICAL ET RÉCIPIENT DE STÉRILISATION À USAGE MÉDICAL

(30) Priorität: 08.12.2015 DE 102015121322
(43) Veröffentlichungstag der Anmeldung: 17.10.2018
(73) Patentinhaber: Aesculap AG, 78532 Tuttlingen (DE)
(72) Erfinder: KOLLER, Tobias, 78532 Tuttlingen (DE)
(74) Vertreter: Hoeger, Stellrecht & Partner Patentanwälte mbB
(86) Internationale Anmeldenummer: PCT/EP2016/079708
(87) Internationale Veröffentlichungsnummer: WO 2017/097689

(56) Entgegenhaltungen:
- EP-A2- 0 412 571
- DE-A1- 3 202 430
- DE-A1-102009 034 992
- DE-A1-102013 111 979
- US-A1- 2012 156 096

## Beschreibung

Die vorliegende Erfindung betrifft eine medizinische Umlaufsperrvorrichtung, umfassend mindestens einen Umlaufkörper und ein Rastelement, wobei der mindestens eine Umlaufkörper eine Zwangsführung und mindestens eine Rastaufnahme für das Rastelement aufweist, und wobei das Rastelement durch die Zwangsführung von einer Ausraststellung in eine Einraststellung an einer ersten Seite der mindestens einen Rastaufnahme in dieselbe und an einer zweiten Seite der mindestens einen Rastaufnahme von der Einraststellung wieder in eine Ausraststellung führbar ist.

Die vorliegende Offenbarung betrifft ferner ein medizinisches Instrument, umfassend eine medizinische Umlaufsperrvorrichtung.

Die Offenbarung betrifft ferner einen medizinischen Sterilisierbehälter, umfassend eine Umlaufsperrvorrichtung.

Eine Umlaufsperrvorrichtung der eingangs genannten Art kommt beispielsweise bei einem chirurgischen Instrument zum Einsatz, das zwei relativ zueinander bewegbare Teile aufweist, deren eines das Rastelement umfasst und deren anderes den mindestens einen Umlaufkörper. Die Teile können relativ zueinander bewegt werden, wodurch das Rastelement in die Rastaufnahme geführt wird. Bei weiterem Bewegen der Teile relativ zueinander in derselben Richtung entrastet das Rastelement aus der Rastaufnahme, und die Teile können in der Gegenrichtung relativ zueinander bewegt werden. Bei diesem Vorgang durchläuft das Rastelement die Zwangsführung und wird von einer Ausraststellung zunächst in die Einraststellung und anschließend wieder in eine Ausraststellung überführt. Ein derartiges Instrument ist beispielsweise in der DE 103 14 072 A1 beschrieben. Bei der Umlaufsperre dieses Instrumentes ist vorgesehen, wie auch bei weiteren chirurgischen Instrumenten, dass die Teile durch einen Benutzer relativ zueinander bewegt werden.

Die DE 10 2009 034 992 A1 beschreibt eine Überwachungseinrichtung für einen Sterilisationsbehälter mit einem Kontrollelement, welches zwischen einer Ruheposition und einer Aktivposition verlagerbar an einem Sterilisationsbehälter gelagert ist und welches nach Durchführung eines Sterilisationsvorganges aus der Ruheposition in die Aktivposition verlagert ist. Die Überwachungseinrichtung umfasst ein Vorschubelement, welches zumindest teilweise aus einer Formgedächtnis-Legierung besteht und eine Umwandlungstemperatur hat, die über der Umgebungstemperatur und dicht bei der Sterilisationstemperatur liegt, welches Vorschubelement das Kontrollelement durch eine Formumwandlung aus der Ruheposition in die Aktivposition verschiebt, wenn das Vorschubelement auf eine oberhalb der Umwandlungstemperatur liegende Temperatur erwärmt wird. Eine Rückhalteeinrichtung ist vorgesehen, die eine Verschiebung des Kontrollelementes aus der Ruheposition in die Aktivposition ermöglicht, eine Verschiebung in der umgekehrten Richtung aus der Aktivposition in die Ruheposition dagegen verhindert.

Aufgabe der vorliegenden Erfindung ist es, eine gattungsgemäße medizinische Umlaufsperreinrichtung bereitzustellen, die die Bewegung von zwei Teilen relativ zueinander ohne Zutun des Benutzers ermöglicht.

Diese Aufgabe wird durch eine erfindungsgemäße Umlaufsperrvorrichtung mit den Merkmalen von Anspruch 1 gelöst.

In die vorliegende Erfindung fließt der Gedanke mit ein, dass durch Bewegung des Rastelementes in der Zwangsführung ein mit diesem gekoppeltes Wirkungselement ebenfalls bewegt werden kann. Zu diesem Zweck umfasst die Umlaufsperrvorrichtung das Kopplungselement. Zur Bewegung des Rastelementes durch die Zwangsführung ist ein mit dem Rastelement wirkverbundenes thermisches Betätigungselement vorgesehen. Thermisch bedingt kann das Betätigungselement von einem ersten Zustand in einen zweiten Zustand und umgekehrt überführt werden. Es ist vorgesehen, dass das Betätigungselement einen Niedertemperaturzustand und einen Hochtemperaturzustand einnehmen kann. Dies gibt die Möglichkeit, dass das Betätigungselement durch eine Temperaturänderung zwischen dem Niedertemperaturzustand und dem Hochtemperaturzustand und umgekehrt wechseln kann. Der erste und der zweite Zustand umfassen ferner einen Zustand größerer Erstreckung und einen Zustand geringerer Erstreckung. Abhängig vom jeweiligen Zustand kann das Betätigungselement aus diesem Grund unterschiedlich erstreckt sein, wodurch eine temperaturbedingte Bewegung des mit dem Betätigungselement wirkverbundenen Rastelementes ausgelöst werden kann. Auf diese Weise kann die Umlaufsperrvorrichtung beispielsweise an einem medizinischen Instrument zum Einsatz kommen, dessen Wirkungselement (erster Teil) bei einer Temperaturänderung am Instrument (zweiter Teil) bewegt werden soll, wobei das Wirkungselement nach zweimaliger entgegengesetzter Temperaturänderung - Erwärmung und Abkühlung oder Abkühlung und Erwärmung - unterschiedliche Stellungen einnehmen kann. Dies wird dadurch erreicht, dass das Rastelement ausgehend von der Ausraststellung in die Einraststellung überführt und zunächst in dieser gehalten werden kann. Das Wirkungselement kann dadurch von einer Ursprungsstellung in eine zweite Stellung bewegt und in dieser gehalten werden. Zum Bewegen des Wirkungselementes wieder zurück in die Ursprungsstellung kann die Umlaufsperrvorrichtung erneut zwei entgegengesetzten Temperaturänderungen unterworfen werden - Erwärmung und Abkühlung oder Abkühlung und Erwärmung - so dass das Rastelement von der Einraststellung aus der Rastaufnahme wieder in eine Ausraststellung überführt wird.

Vorzugsweise kann die Umlaufsperrvorrichtung von einem medizinischen Sterilisierbehälter mit einer Ventileinrichtung umfasst sein, wobei das Wirkungselement den Ventilkörper der Ventileinrichtung bildet. Dies ermöglicht es insbesondere, ausgehend von einem Schließzustand einen Öffnungszustand und einen erneuten Schließzustand der Ventileinrichtung nur nach mindestens zwei aufeinanderfolgenden Erwärmungs- und Abkühlphasen durchzuführen.

Es kann vorgesehen sein, dass der erste Zustand der Niedertemperaturzustand ist und der zweite Zustand der Hochtemperaturzustand. Durch Erwärmen und Abkühlen kann das Rastelement von der Ausraststellung in die Einraststellung und durch nochmaliges Erwärmen und Abkühlen von der Einraststellung in die Ausraststellung gebracht werden.

Alternativ kann vorgesehen sein, dass der erste Zustand der Hochtemperaturzustand ist und der zweite Zustand der Niedertemperaturzustand. Durch Abkühlen und Erwärmen kann das Rastelement von der Ausraststellung in die Einraststellung und durch nochmaliges Abkühlen und Erwärmen von der Einraststellung in die Ausraststellung gebracht werden.

Weiter kann vorgesehen sein, dass der erste Zustand der Zustand geringerer Erstreckung ist und der zweite Zustand der Zustand größerer Erstreckung. Durch Überführen des Betätigungselementes von dem ersten in den zweiten Zustand kann das Betätigungselement mit größerer Erstreckung versehen werden. Durch Überführen vom zweiten in den ersten Zustand kann die Erstreckung des Rastelementes verringert werden. Es kann vorgesehen sein, dass zum Bewegen des Rastelementes in letzterem Fall ein von der Umlaufsperrvorrichtung umfasstes Stellelement mit dem Rastelement wirkverbunden ist. Beispielsweise greift das Stellelement am Kopplungselement an, um das Rastelement in die Einraststellung bzw. wieder in die Ausraststellung zu bewegen.

Alternativ kann vorgesehen sein, dass der erste Zustand der Zustand größerer Erstreckung ist und der zweite Zustand der Zustand geringerer Erstreckung. Dabei kann vorgesehen sein, dass beim Überführen des Betätigungselementes vom ersten Zustand in den zweiten Zustand unter Verringerung von dessen Erstreckung ein Stellelement mit dem Rastelement wirkverbunden ist, um dieses zu bewegen. Beispielsweise greift das Stellelement am Kopplungselement an. Beim Überführen des Betätigungselementes vom zweiten Zustand in den ersten Zustand und unter Vergrößerung der Erstreckung des Betätigungselements kann das Betätigungselement das Rastelement in die Einraststellung bzw. wieder in die Ausraststellung bewegen.

Bei einer vorteilhaften Ausführungsform der Umlaufsperrvorrichtung ist der erste Zustand der Niedertemperaturzustand geringerer Erstreckung des Betätigungselementes und der zweite Zustand der Hochtemperaturzustand größerer Erstreckung des Betätigungselementes.

Als vorteilhaft erweist es sich, wenn das Betätigungselement aus einem Phasenumwandlungsmaterial besteht oder ein solches umfasst, das im ersten Zustand und im zweiten Zustand unterschiedliche Phasen einnimmt. Das Phasenumwandlungsmaterial kann ein Phasenwechselmaterial sein, wie zum Beispiel ein Latentwärmespeichermaterial, oder das nachfolgend genannte Formgedächtnismaterial.

Vorzugsweise besteht das Betätigungselement aus einem Formgedächtnismaterial oder umfasst ein solches.

Vorteilhafterweise ist das Formgedächtnismaterial ein Zweiweg-Formgedächtnismaterial. Das Betätigungselement kann sowohl im Hochtemperaturzustand als auch im Niedertemperaturzustand unterschiedliche, eingeprägte Formen aufweisen, in denen das Betätigungselement unterschiedlich große Erstreckungen aufweist. Durch eine Temperaturänderung kann das Betätigungselement von der einen in die jeweils andere Form zur Änderung der Erstreckung überführt werden.

Das Formgedächtnismaterial ist vorzugsweise ein Formgedächtnismetall. "Formgedächtnismetall" schließt vorliegend eine Legierung von Metallen ein.

Beispielsweise ist das Formgedächtnismetall Nitinol, wobei auch andere Metalllegierungen denkbar sind.

Das Formgedächtnismaterial kann beispielsweise auch ein Formgedächtnispolymer sein.

Das Betätigungselement kann als Federelement ausgestaltet sein, zum Beispiel als Druckfeder oder alternativ als Zugfeder.

Beispielsweise ist das Betätigungselement schraubenförmig und umgibt das Kopplungselement und/oder das Wirkungselement.

Als günstig erweist es sich, wenn die Umlaufsperrvorrichtung ein Stellelement umfasst, das mit dem Rastelement in Wirkverbindung steht und dieses bewegt, wenn das Betätigungselement vom Zustand größerer Erstreckung in den Zustand geringerer Erstreckung überführt wird. Wird das Betätigungselement vom Zustand geringerer in den Zustand größerer Erstreckung überführt, kann es Arbeit zum Bewegen des Rastelementes verrichten. Das Stellelement kann in entsprechender Weise Arbeit am Rastelement verrichten, wenn das Betätigungselement vom Zustand größerer in den Zustand geringerer Erstreckung überführt wird.

Das Stellelement kann als Federelement ausgestaltet sein, bei dem es sich zum Beispiel um eine Druckfeder handeln kann. Alternativ ist denkbar, dass das Stellelement als Zugfeder ausgestaltet ist. Das Federelement kann schraubenförmig sein und das Kopplungselement umgeben.

Von Vorteil ist es, wenn die Umlaufsperrvorrichtung mindestens ein am Kopplungselement festgelegtes Anschlagelement für das Betätigungselement und/oder für das Stellelement aufweist oder bildet. Eine Kraft des Betätigungselementes und/oder des Stellelementes zum Bewegen des Kopplungselementes und dadurch des Rastelementes kann am Anschlagelement angreifen. Das Anschlagelement ist insbesondere ein relativ zu einem die Umlaufsperrvorrichtung umfassenden Instrument beweglicher Anschlag. Das Betätigungselement und/oder das Stellelement können sich am mindestens einen Anschlagelement abstützen.

Günstigerweise ist ein gemeinsames Anschlagelement vorgesehen, wobei das Betätigungselement und das Stellelement mit Kräften in einander entgegengesetzten Richtungen am Anschlagelement angreifen.

Beispielsweise sind das Betätigungselement und das Stellelement an einander gegenüberliegenden Seiten des Anschlagelementes angeordnet.

Von Vorteil ist es, wenn die Umlaufsperrvorrichtung mindestens ein Anschlagelement für das Betätigungselement aufweist oder bildet, welches mindestens eine Anschlagelement an einem die Umlaufsperrvorrichtung umfassenden Instrument festgelegt oder festlegbar ist. Das Anschlagelement kann dadurch insbesondere ortsfest am Instrument sein und eine Kraft aufnehmen (beispielsweise indem sich das Betätigungselement am Anschlagelement abstützt), deren Gegenkraft vom Betätigungselement auf das zuvor genannte Anschlagelement zum Bewegen des Kopplungselementes ausgeübt wird.

Günstig ist es, wenn die Umlaufsperrvorrichtung mindestens ein Anschlagelement für das Stellelement aufweist oder bildet, welches mindestens eine Anschlagelement an einem die Umlaufsperrvorrichtung umfassenden Instrument festgelegt oder festlegbar ist. Das Anschlagelement kann dadurch insbesondere ortsfest zum Instrument sein und eine Gegenkraft zu derjenigen Kraft aufnehmen (beispielsweise indem sich das Stellelement am Anschlagelement abstützt), die vom Stellelement auf das erstgenannte Anschlagelement ausgeübt wird.

Der mindestens eine Umlaufkörper kann ein Lagerelement aufweisen zur drehbaren und/oder verschiebbaren Lagerung an einem die Umlaufsperrvorrichtung umfassenden Instrument.

Das Kopplungselement kann mindestens ein Lagerelement aufweisen zur verschiebbaren und/oder drehbaren Lagerung an einem die Umlaufsperrvorrichtung umfassenden Instrument.

Das Wirkungselement kann ein Ventilkörper einer Ventileinrichtung sein oder einen solchen umfassen. Dabei handelt es sich zum Beispiel um die Ventileinrichtung eines medizinischen Sterilisierbehälters.

Das Rastelement kann über das Kopplungselement starr mit dem Wirkungselement gekoppelt sein.

Günstigerweise ist das Kopplungselement einstückig mit dem Rastelement und/oder mit dem Wirkungselement gebildet.

Das Kopplungselement kann stangenförmig sein.

Die Zwangsführung ist beispielsweise eine Kulissenführung.

Die mindestens eine Rastaufnahme ist beispielsweise als Rastrücksprung am mindestens einen Umlaufkörper ausgebildet.

Bei einer vorteilhaften Ausführungsform der Umlaufsperrvorrichtung weist die Zwangsführung eine Rampe für das Rastelement auf, über welche dieses beim Überführen von der Ausraststellung in die Einraststellung gleitet, wobei die Zwangsführung ein das Rastelement nach dem Übergleiten zurückhaltendes Anschlagglied umfasst, an dem eine das Rastelement in die Rastaufnahme führende Steuerfläche angeordnet ist. Das Rastelement kann über die Rampe in der Zwangsführung bewegt werden, wenn das Betätigungselement vom ersten in den zweiten Zustand überführt wird. Wird das Betätigungselement vom zweiten in den ersten Zustand überführt, kann das Rastelement durch das Anschlagglied zurückgehalten werden und über die Steuerfläche in die Einraststellung in der Rastaufnahme geführt werden.

Die Zwangsführung weist vorzugsweise einen die Rampe bildenden Eingangskanal für das Rastelement auf, um eine zuverlässige Führung des Rastelementes zu ermöglichen.

Am Eingangskanal kann eine Steuerfläche für das Rastelement angeordnet sein, um dieses in den Eingangskanal zu führen. Es kann vorgesehen sein, dass zu diesem Zweck der Umlaufkörper am Lagerelement relativ zu dem Instrument bewegt und insbesondere verschwenkt wird, wenn das Rastelement die Steuerfläche kontaktiert.

Günstigerweise weist die Zwangsführung eine Steuerfläche auf, welche das Rastelement beim Überführen von der Einraststellung in die Ausraststellung kontaktiert, um den mindestens einen Umlaufkörper an einem Lagerelement relativ zum Instrument zu bewegen und insbesondere zu verschwenken. Das Rastelement kann durch Überführen des Betätigungselementes vom ersten in den zweiten Zustand bewegt werden und die Steuerfläche kontaktieren. Dies erlaubt es zum Beispiel, den Umlaufkörper am Lagerelement zu bewegen, insbesondere zu verschwenken. Beispielsweise wird das Rastelement dadurch in einen Ausgangskanal der Zwangsführung überführt.

Dementsprechend kann die Zwangsführung einen Ausgangskanal für das Rastelement aufweisen, an dessen Anfang die Steuerfläche angeordnet ist.

Die Zwangsführung kann eine Rampe aufweisen, beispielsweise am Ausgangskanal. Es kann vorgesehen sein, dass das Rastelement beim Überführen von der Einraststellung in die Ausraststellung über die Rampe gleitet, wobei die Zwangsführung ein das Rastelement nach dem Übergleiten zurückhaltendes Anschlagglied umfasst, um einen Eintritt in die Zwangsführung in entgegengesetzter, "falscher" Richtung zu vermeiden.

Es kann vorgesehen sein, dass der mindestens eine Umlaufkörper genau eine Rastaufnahme aufweist, ausgehend von der das Rastelement in die Ausraststellung überführbar ist. Beispielsweise wird zu diesem Zweck das Rastelement unter der Wirkung des Stellelementes von der Einraststellung zurück in die Ausraststellung überführt.

Bei einer andersartigen vorteilhaften Ausführungsform der Umlaufsperrvorrichtung kann vorgesehen sein, dass der mindestens eine Umlaufkörper mindestens eine weitere Rastaufnahme für das Rastelement aufweist, in der dieses eine weitere Einraststellung einnehmen kann, wobei zum Überführen des Rastelementes von der Einraststellung in die weitere Einraststellung das Betätigungselement vom zweiten Zustand in den ersten Zustand und wieder in den zweiten Zustand überführbar ist und dadurch das Rastelement in der Zwangsführung bewegbar ist. Dies gibt zum Beispiel die Möglichkeit, dass das Wirkungselement erst nach drei Zyklen einer Temperaturänderung (dreimalige Erwärmung und Abkühlung oder Abkühlung und Erwärmung) und zwischenzeitlichem zweimaligen Verrasten des Rastelementes in der Rastaufnahme wieder in die Ausgangsposition zurück bewegt wird.

Entsprechend der vorstehend genannten Ausführungsform kann auch vorgesehen sein, dass der Umlaufkörper mehr als zwei Rastaufnahmen für das Rastelement aufweist, wobei ausgehend von einer Rastaufnahme das Rastelement jeweils durch Überführen des Betätigungselementes vom ersten in den zweiten Zustand und umgekehrt in die nächste Rastaufnahme überführt werden kann.

Eine vorteilhafte Ausführungsform der Umlaufsperrvorrichtung kann eine Mehrzahl von an einem gemeinsamen Halteteil festgelegten oder gebildeten Umlaufkörpern aufweisen, wobei ein jeweiliger Ausgang einer Zwangsführung eines Umlaufkörpers in einen jeweiligen Eingang einer Zwangsführung eines benachbarten Umlaufkörpers mündet oder diesem benachbart ist. Die Umlaufkörper sind beispielsweise an einem am Instrument drehbaren Halteteil gehalten und radial bezüglich eines Lagerelementes ausgerichtet, das die Drehachse definiert.

Die zuletzt erwähnte vorteilhafte Ausführungsform ist insbesondere von Vorteil, wenn die Umlaufsperrvorrichtung ein Zählwerk umfasst, das mit dem Halteteil in Wirkverbindung steht und einen jeweiligen Umlauf des Rastelementes an den Umlaufkörpern zählt. Dies erlaubt es zum Beispiel, Zyklen von Temperaturänderungen (Erwärmung und Abkühlung oder Abkühlung und Erwärmung) zu zählen. Hierbei ist beispielsweise vorgesehen, dass bei einer Anzahl von n Umlaufkörpern mit jeweils einer Rastaufnahme 2n Zyklen von Temperaturänderungen zu absolvieren sind, bevor das Zählwerk um einen Schritt voranschreitet. Das Zählwerk kann mechanisch oder elektronisch ausgestaltet sein und eine Anzeige aufweisen.

Wie eingangs erwähnt, betrifft die vorliegende Offenbarung auch ein medizinisches Instrument. Ein Instrument gemäß der Offenbarung umfasst eine Umlaufsperrvorrichtung der vorstehend genannten Art.

Das Instrument kann insbesondere ein medizinischer Sterilisierbehälter sein.

Die vorliegende Offenbarung betrifft dementsprechend auch einen medizinischen Sterilisierbehälter. Ein Sterilisierbehälter umfasst eine Umlaufsperrvorrichtung der vorstehend genannten Art, wobei der Sterilisierbehälter eine Ventileinrichtung mit einer in einer Behälterwand gebildeten Ventilöffnung und einem Ventilkörper aufweist, wobei der Ventilkörper das Wirkungselement der Umlaufsperrvorrichtung ist.

Der Ventilkörper kann zum Beispiel durch Erwärmung von einer die Ventilöffnung überdeckenden Schließstellung durch das Betätigungselement bewegt werden und bei erneuter Abkühlung unter Freigabe der Ventilöffnung im Abstand zu dieser in einer Offenstellung angeordnet sein, wobei das Rastelement in der Rastaufnahme verrastet. Bei erneuter Erwärmung und darauffolgender Abkühlung kann der Ventilkörper wieder von der ventilöffnungsfernen Offenstellung in eine Schließstellung überführt werden, in der der Ventilkörper die Ventilöffnung verschließt. Die Offenbarung bietet auf diese Weise die Möglichkeit, einen Öffnungs- und Schließzustand der Ventileinrichtung nur nach mindestens zwei aufeinanderfolgenden Erwärmungs- und Abkühlvorgängen durchzuführen. Weist der Umlaufkörper mehr als zwei Rastaufnahmen auf, wie vorstehend erläutert, ist auch denkbar, dass mehr als zwei Erwärmungs- und Abkühlvorgänge durchzuführen sind. Bei einem derartigen Sterilisierbehälter ist zum Beispiel vorgesehen, dass über die Ventilöffnung Kondensat, das bei einem Dampfsterilisiervorgang anfällt, aus dem Behälterinnenraum abgeleitet werden soll. Hierbei kann die Umlaufsperrvorrichtung mit einer Ventilöffnung vergleichsweise geringen Querschnitts zum Einsatz kommen, wobei auftretendes Kondensat durch Halten des Ventilkörpers in der Offenstellung nach einer Mehrzahl von Temperaturänderungen sicher abgeleitet werden kann. Günstigerweise verschließt der Ventilkörper die Ventilöffnung in der Ausraststellung des Rastelementes und gibt diese in der Einraststellung des Rastelementes frei.

Die nachfolgende Beschreibung bevorzugter Ausführungsformen der Erfindung dient im Zusammenhang mit der Zeichnung der näheren Erläuterung der Erfindung. Es zeigen:
- Figur 1:: schematisch einen medizinischen Sterilisierbehälter, umfassend eine erfindungsgemäße medizinische Umlaufsperrvorrichtung ;
- Figur 2:: schematisch die Umlaufsperrvorrichtung des Sterilisierbehälters aus Figur 1 mit einem eine Ausraststellung einnehmenden Rastelement und einem einen ersten Zustand einnehmenden Betätigungselement;
- Figur 3:: eine Darstellung entsprechend Figur 2, wobei das Betätigungselement einen zweiten Zustand einnimmt und abschnittsweise auch eine Behälterwand des Sterilisierbehälters gezeigt ist;
- Figur 4:: eine Darstellung entsprechend Figur 2, wobei das Rastelement eine Einraststellung einnimmt und das Betätigungselement einen ersten Zustand;
- Figur 5:: eine Darstellung entsprechend Figur 2, wobei das Betätigungselement einen zweiten Zustand einnimmt;

- Figur 6:: einen Umlaufkörper mit einer Zwangsführung für das Rastelement der Umlaufsperrvorrichtung und
- Figur 7:: eine schematische Teildarstellung einer weiteren bevorzugten Ausführungsform einer Umlaufsperrvorrichtung.

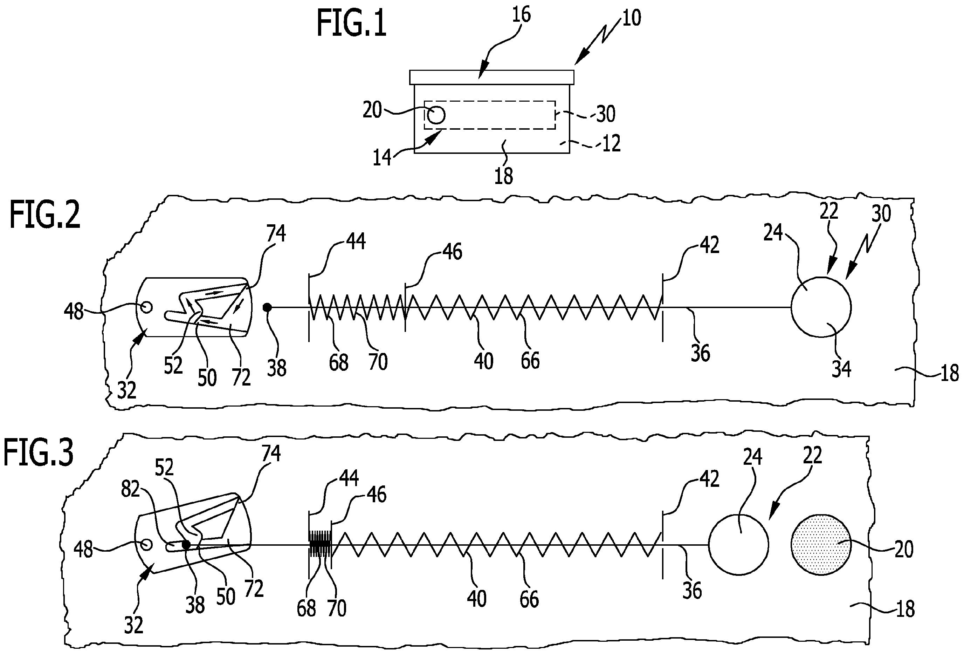

Figur 1 zeigt eine bevorzugte Ausführungsform eines medizinischen Sterilisierbehälters, der mit dem Bezugszeichen 10 belegt ist. Der Sterilisierbehälter 10 wird vorliegend als medizinisches Instrument angesehen.

Der Sterilisierbehälter 10 definiert einen Behälterinnenraum 12, in dem zu sterilisierende chirurgische Instrumente positioniert werden können. Der Behälterinnenraum 12 wird gebildet durch eine Sterilisierbehälterwanne 14 und einen auf diese aufgesetzten Sterilisierbehälterdeckel 16. Der Sterilisierbehälter 10 umfasst eine als Ganzes mit 18 bezeichnete Behälterwand. Bei der Behälterwand kann es sich um eine Wandung der Sterilisierbehälterwanne 14 (zum Beispiel die Bodenwand oder eine Seitenwand) handeln oder um eine Wandung des Sterilisierbehälterdeckels 16.

In der Behälterwand 18, vorliegend beispielhaft in einer Seitenwand der Sterilisierbehälterwanne 14, ist eine Ventilöffnung 20 gebildet. Die Ventilöffnung 20 ist in Figur 1 und auch in den Figuren 3 bis 5 schematisch dargestellt. Die Figuren 2 bis 5 und 7 zeigen die Behälterwand 18 abschnittsweise.

Die Ventilöffnung 20 ist Bestandteil einer Ventileinrichtung 22 des Sterilisierbehälters 10, die ferner einen Ventilkörper 24 aufweist. Der Ventilkörper 24, und damit die Ventileinrichtung 22, können eine Schließstellung einnehmen, in der der Ventilkörper 24 die Ventilöffnung 20 dichtend verschließt (Figur 2). In der Schließstellung des Ventilkörpers 24 ist der Sterilisierbehälter 10 dicht verschlossen.

Der Ventilkörper 24 und damit die Ventileinrichtung 22 können ferner eine Öffnungsstellung einnehmen, in der der Ventilkörper 24 von einem Ventilsitz an der Ventilöffnung 20 angehoben ist (zum Beispiel durch Verschieben) und im Abstand zur Ventilöffnung 20 angeordnet ist. Die Ventilöffnung 20 ist dadurch freigegeben (Figur 3).

Beim Dampfsterilisieren von Instrumenten wird Dampf in den Sterilisierbehälter 10 eingeleitet, und der Sterilisierbehälter 10 wird Temperaturänderungen unterworfen, nämlich in aufeinanderfolgenden Zyklen wiederholt erwärmt und abgekühlt. Beim Sterilisieren tritt Kondensat auf, das bevorzugt aus dem Behälterinnenraum 12 am Ende des Sterilisiervorganges abzuleiten ist. Zu diesem Zweck kann die Ventileinrichtung 22 auf nachfolgend erläuterte Weise in die Offenstellung überführt werden. Bei freigegebener Ventilöffnung 20 kann das Kondensat durch die Ventilöffnung 20 hindurch aus dem Behälterinnenraum 12 abgeleitet werden.

Um den Ventilkörper 24 in die Öffnungsstellung und in die Schließstellung zu überführen, muss dieser relativ zur Ventilöffnung 20 bewegt werden. Zu diesem Zweck weist der Sterilisierbehälter 10 eine mit dem Bezugszeichen 30 belegte vorteilhafte Ausführungsform einer erfindungsgemäßen Umlaufsperrvorrichtung auf. Figur 1 zeigt mit gestrichelten Linien die Kontur der Umlaufsperrvorrichtung 30, die ferner in den Figuren 2 bis 5 schematisch gezeigt ist.

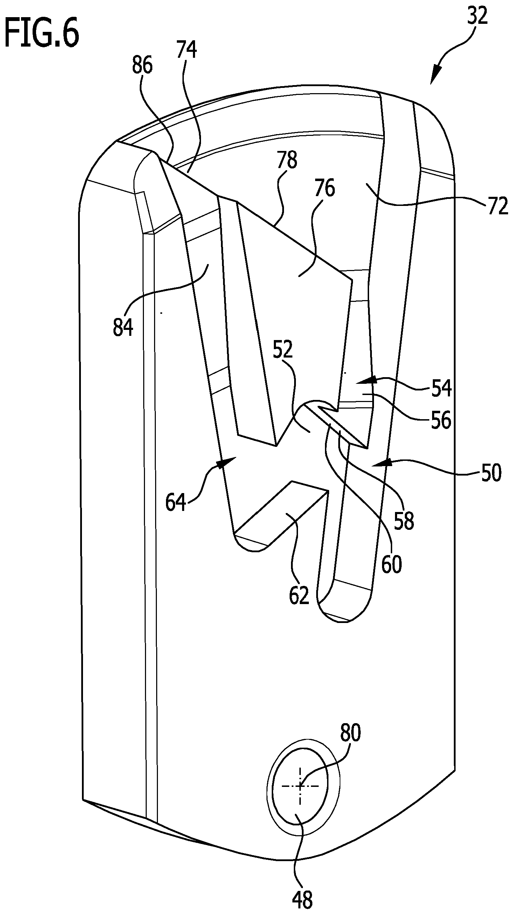

Die Umlaufsperrvorrichtung 30 umfasst einen Umlaufkörper 32, der in Figur 6 gesondert dargestellt ist. Ferner umfasst die Umlaufsperrvorrichtung 30 ein Wirkungselement 34, vorliegend den Ventilkörper 24. Die Umlaufsperrvorrichtung 30 weist ferner ein Kopplungselement 36, ein Rastelement 38, ein Betätigungselement 40, ein Stellelement 68 sowie Anschlagelemente 42, 44 und 46 auf.

Der Umlaufkörper 32 umfasst ein Lagerelement 48 sowie eine Zwangsführung 50 und eine Rastaufnahme 52 für das Rastelement 38. Die Zwangsführung 50 ist eine Kulissenführung und umfasst einen Eingangskanal 54 für das Rastelement 38, eine Rampe 56 mit einem Anschlagglied 58, eine Steuerfläche 60, eine weitere Steuerfläche 62 und einen Ausgangskanal 64 für das Rastelement 38. Der Eingangskanal 54 und der Ausgangskanal 64 sind auf einander gegenüberliegenden Seiten der Rastaufnahme 52 angeordnet.

Der Ventilkörper 24 ist über das Kopplungselement 36 vorzugsweise starr mit dem Rastelement 38 gekoppelt. Das Rastelement 38 und der Ventilkörper 24 sind auf einander gegenüberliegenden Seiten des Kopplungselementes 36 angeordnet und vorzugsweise einstückig mit diesem verbunden. Das Kopplungselement 36 ist vorliegend stangenförmig. Das Rastelement 38 ist beispielsweise ein Rastvorsprung.

Das Kopplungselement 36 ist an der Behälterwand 18 beweglich und insbesondere verschieblich gelagert. Durch Verschieben des Kopplungselementes 36 an der Behälterwand 18 kann der Ventilkörper 24 von der Ventilöffnung 20 weg und auf diese zu geschoben werden.

Die Anschlagelemente 42 und 44 sind an der Behälterwand 18 festgelegt. Dadurch sind beide Anschlagelemente 42, 44 ortsfest zu dem die Umlaufsperrvorrichtung 30 umfassenden Sterilisierbehälter 10. Das Kopplungselement 36 durchgreift im vorliegenden Fall die Anschlagelemente 42 und 44. Der Ventilkörper 24 und das Rastelement 38 sind auf einander abgewandten Seiten des Anschlagelementes 42 einerseits bzw. des Anschlagelementes 44 andererseits positioniert.

Das Anschlagelement 46 ist am Kopplungselement 36 festgelegt und beispielsweise einstückig mit diesem verbunden. Das Anschlagelement 46 ist dadurch beweglich relativ zur Behälterwand 18 und insbesondere beweglich an dem die Umlaufsperrvorrichtung 30 umfassenden Sterilisierbehälter 10. Umgekehrt können durch eine Bewegung des Anschlagelementes 46 der Ventilkörper 24 und das Rastelement 38 verschoben werden. Das Anschlagelement 46 ist zwischen den Anschlagelementen 42 und 44 angeordnet.

Zwischen dem Anschlagelement 42 und dem Anschlagelement 46 ist das Betätigungselement 40 positioniert. Das Betätigungselement 40 ist vorliegend thermisch betätigbar und ausgestaltet als Federelement 66, insbesondere als Druckfeder. Andersartige Ausgestaltungen sind selbstverständlich denkbar.

Das Betätigungselement 40 umgibt das Kopplungselement 36 und kann sich mit einer ersten Seite am Anschlagelement 42 abstützen und mit einer zweiten Seite am Anschlagelement 46.

Das Betätigungselement 40 ist aus einem Phasenumwandlungsmaterial gefertigt, bei dem es sich vorliegend um ein Formgedächtnismaterial handelt. Das Formgedächtnismaterial ist insbesondere ein Zweiweg-Formgedächtnismaterial. Als Formgedächtnismaterial kommt ein Formgedächtnismetall zum Einsatz, vorzugsweise Nitinol (Nickel-Titan-Legierung).

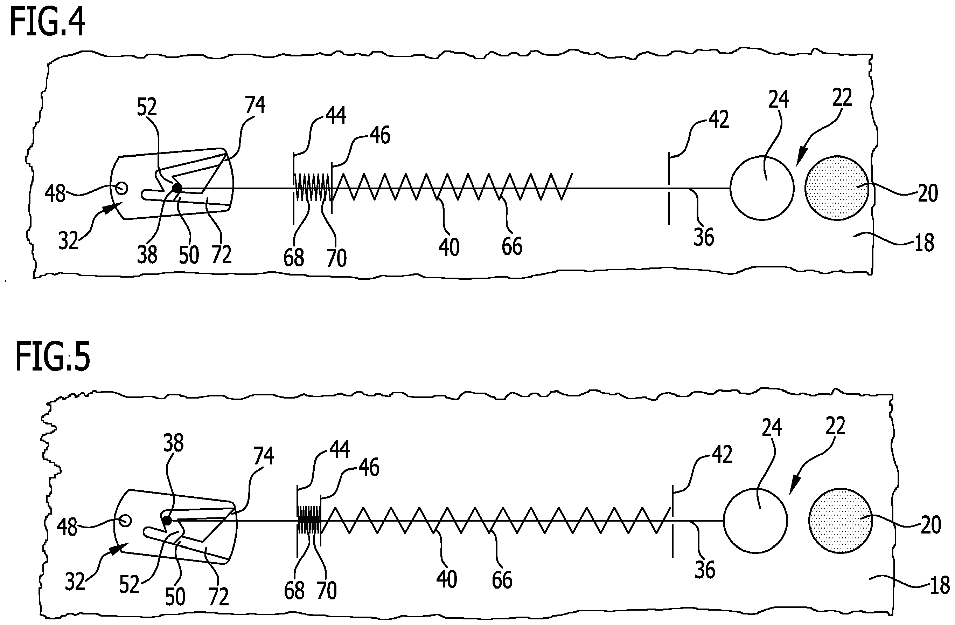

Das Betätigungselement 40 kann aufgrund des Zweiweg-Formgedächtnismaterials in zwei unterschiedlichen Zuständen verschiedenartige, jeweils eingeprägte Formen einnehmen. In einem ersten Zustand, einem Niedertemperaturzustand, weist das Betätigungselement 40 einen Zustand geringerer Erstreckung auf. Die Figuren 2 und 4 zeigen das Betätigungselement 40 im Niedertemperaturzustand, dem Zustand geringerer Erstreckung.

Durch Temperaturerhöhung kann das Betätigungselement 40 in einen zweiten Zustand überführt werden. Der zweite Zustand ist ein Hochtemperaturzustand, in dem das Betätigungselement 40 einen Zustand größerer Erstreckung aufweist. Die Figuren 3 und 5 zeigen das Betätigungselement 40 im Hochtemperaturzustand, dem Zustand größerer Erstreckung.

Im Niedertemperaturzustand ist die Erstreckung des Betätigungselementes 40 geringer als im Hochtemperaturzustand. Unabhängig vom Zustand ist das Betätigungselement 40 schraubenförmig.

Durch Erwärmung kann das Betätigungselement 40 vom Niedertemperaturzustand in den Hochtemperaturzustand überführt und dadurch in seiner Erstreckung vergrößert werden. Umgekehrt kann das Betätigungselement 40 ausgehend vom Hochtemperaturzustand durch Abkühlung in den Niedertemperaturzustand überführt und dadurch in seiner Erstreckung verringert werden. Der Niedertemperaturzustand und der Hochtemperaturzustand werden durch unterschiedliche Phasen des Nitinol-Phasenumwandlungsmaterials eingenommen.

Zwischen dem Anschlagelement 44 und dem Anschlagelement 46 ist das Stellelement 68 der Umlaufsperrvorrichtung 30 angeordnet. Das Stellelement 68 ist ausgestaltet als Federelement 70, insbesondere als Schraubenfeder. Das Federelement 70 umgibt das Kopplungselement 36. Mit einer ersten Seite kann sich das Stellelement 68 am Anschlagelement 44 abstützen und mit einer zweiten Seite am Anschlagelement 46.

Kräfte, die vom Betätigungselement 40 einerseits und vom Stellelement 68 andererseits auf das Anschlagelement 46 gerichtet sind, sind einander entgegengesetzt. Dementsprechend ist auch das Stellelement 68 eine Druckfeder.

Das Stellelement 68 ist vorliegend ein Rückstellelement zum Bewegen des Kopplungselementes 36 und damit des Rastelementes 38, wenn das Betätigungselement 40 vom Hochtemperaturzustand in den Niedertemperaturzustand überführt wird.

Der Umlaufkörper 32 weist eine dem Kopplungselement 36 zugewandte erste Seite auf und eine dem Kopplungselement 36 abgewandte zweite Seite. An der ersten Seite ist ein Eingang 72 der Zwangsführung 50 angeordnet, über den das Rastelement 38 in den Eingangskanal 54 eintreten kann. Ferner ist an der ersten Seite ein Ausgang 74 der Zwangsführung 50 angeordnet, an dem das Rastelement 38 aus dem Ausgangskanal 64 austreten kann.

Zwischen dem Eingang 72 und dem Ausgang 74 ist ein Vorsprung 76 am Umlaufkörper 32 gebildet. Der Vorsprung 76 weist eine Steuerfläche 78 auf. Die Steuerfläche 78 umfasst eine Neigung relativ zur vom Kopplungselement 36 definierten Richtung. Die Neigung der Steuerfläche 78 verläuft in Eintrittsrichtung des Kopplungselementes 36 in den Eingangskanal 54.

Die Rastaufnahme 52 ist ein Rastrücksprung an der dem Kopplungselement 36 abgewandten Seite des Vorsprunges 76.

An der zweiten Seite des Umlaufkörpers 32 ist das Lagerelement 48 angeordnet. Das Lagerelement 48 definiert eine Drehachse 80. Der Umlaufkörper 32 ist relativ zur Behälterwand 18 am Lagerelement 48 um die Drehachse 80 verschwenkbar.

Aufgrund des thermisch betätigbaren Betätigungselementes 40 kann beim Sterilisierbehälter 10 die Ventilöffnung 20 ausgehend von der Schließstellung des Ventilkörpers 24 freigegeben und wieder verschlossen werden, indem der Sterilisierbehälter 10 beim Sterilisiervorgang mehreren Zyklen, nämlich zwei, von Temperaturänderungen unterworfen wird. Dabei wird die Ventilöffnung 20 unter Erwärmung freigegeben, verbleibt bei Abkühlung im freigegebenen Zustand, und erst nach erneuter Erwärmung und darauffolgender Abkühlung wird die Ventilöffnung 20 wieder verschlossen. Zu diesem Zweck kommt die Umlaufsperrvorrichtung 30 zum Einsatz, was nachfolgend unter Verweise insbesondere auf die Figuren 2 bis 5 erläutert wird.

Es wird angenommen, dass der Ventilkörper 24 anfänglich den Schließzustand einnimmt und die Ventilöffnung 20 verschließt. Die Temperatur ist niedrig, und das Betätigungselement 40 nimmt den Niedertemperaturzustand geringerer Erstreckung (erster Zustand) ein. Das Rastelement 38 ist außerhalb der Zwangsführung 50 angeordnet und beispielsweise der Steuerfläche 78 gegenüberliegend positioniert. Das Betätigungselement 40 und das Stellelement 68 sind so bemessen, dass sie die Anschlagelemente 42 und 46 bzw. 46 und 44 kontaktieren. Dies ist in Figur 2 gezeigt.

Durch Erwärmung von der niedrigeren Temperatur auf eine höhere Temperatur wird das Betätigungselement 40 in den Hochtemperaturzustand größerer Erstreckung (zweiter Zustand) überführt. Das Betätigungselement 40 dehnt sich aus und stützt sich weiterhin an den Anschlagelementen 42 und 46 ab. Das Anschlagelement 46 und damit das Kopplungselement 36 werden relativ zur Behälterwand 18 verschoben. Dadurch wird auch der Ventilkörper 24 verschoben. Der Ventilkörper 24 ist zur Freigabe von der Ventilöffnung 20 abgehoben. Kondensat kann durch die Ventilöffnung 20 aus dem Behälterinnenraum 12 austreten.

Durch die Kopplung des Betätigungselementes 40 mit dem Rastelement 38 wird auch das Rastelement 38 verschoben. Das Rastelement 38 kontaktiert die Steuerfläche 78, was zur Verschwenkung des Umlaufkörpers 32 um die Drehachse 80 führt. Das Rastelement 38 kann in den Eingangskanal 54 eintreten und die Rampe 56 entlang gleiten. Nach dem Übergleiten der Rampe 56 ist das Rastelement 38 in einer sackgassenartigen Ausnehmung 82 des Eingangskanals 54 positioniert. Dies ist in Figur 3 dargestellt.

Das Verschieben des Anschlagelementes 46 erfolgt unter Kompression des sich an diesem abstützenden Stellelementes 68.

Bei Abkühlung kann das Betätigungselement 40 vom Hochtemperaturzustand größerer Erstreckung in den Niedertemperaturzustand geringerer Erstreckung überführt werden und sich wieder verkürzen. Dadurch ist das Betätigungselement 40 nicht mehr zwischen den Anschlagelementen 42 und 46 gespannt. Das Stellelement 68 kann das Anschlagelement 46 unter Vorspannung verschieben und damit das Kopplungselement 36 zusammen mit dem Rastelement 38. Das Rastelement 38 kontaktiert das Anschlagglied 58 und wird über die daran angeordnete Steuerfläche 60 in die Rastaufnahme 52 geführt.

In der Rastaufnahme 52 nimmt das Rastelement 38 eine Einraststellung ein, wenn das Betätigungselement 40 wieder den ersten Zustand einnimmt. In die Einraststellung wurde das Rastelement 38 somit ausgehend von der Ausraststellung überführt, die es bei Einnahme des ersten Zustandes durch das Betätigungselement 40 eingenommen hat.

Der Ventilkörper 24 bleibt auch nach Abkühlung von der Ventilöffnung 20 abgehoben, so dass weiterhin Kondensat durch die Ventilöffnung 20 aus dem Behälterinnenraum 12 abgeleitet werden kann (Figur 4).

Anschließend kann das Betätigungselement 40 bei erneuter Temperaturerhöhung von dem Niedertemperaturzustand geringerer Erstreckung abermals in den Hochtemperaturzustand größerer Erstreckung überführt werden. Das Betätigungselement 40 verschiebt dadurch erneut das Kopplungselement 36 und das Rastelement 38 durch Abstützen an den Anschlagelementen 42 und 46. Das Rastelement 38 wird aus der Einraststellung in der Rastaufnahme 52 angehoben und kann die Steuerfläche 62 eingangsseitig des Ausgangskanals 64 kontaktieren. Der Umlaufkörper 32 wird um die Drehachse 80 relativ zur Behälterwand 18 verschwenkt, so dass das Rastelement 38 am Anfang des Ausgangskanals 64 positioniert ist.

Beim Verschieben des Kopplungselementes 36 wird das Stellelement 68 erneut komprimiert. Der Ventilkörper 24 bleibt weiterhin von der Ventilöffnung 20 angehoben (Figur 5).

Anschließend kann das Betätigungselement 40 bei einer Verringerung der Temperatur erneut vom Hochtemperaturzustand größerer Erstreckung in den Niedertemperaturzustand geringerer Erstreckung überführt werden. Das Betätigungselement 40 liegt nicht mehr an beiden Anschlagelementen 42 und 46 an. Unter der vorspannenden Wirkung des Stellelementes 68 wird das Anschlagelement 46 verschoben. Dadurch werden auch das Kopplungselement 36 und das Rastelement 38 verschoben.

Das Rastelement 38 wird durch den Ausgangskanal 64 geführt. Der Ausgangskanal 64 weist eine Rampe 84 auf, über die das Rastelement 38 gleitet. Ausgangsseitig an der Rampe 84 ist ein Anschlagglied 86 im Bereich des Ausgangs 74 angeordnet. Nach dem Austreten des Rastelementes 38 aus dem Ausgangskanal 64 und damit der Zwangsführung 50 ist durch das Anschlagglied 86 sichergestellt, dass das Rastelement 38 bei einem erneuten Temperaturänderungszyklus nicht in den Ausgangskanal 64 eintritt. Zu diesem Zweck ist es von Vorteil, wenn das Anschlagglied 86 als Bestandteil oder Verlängerung der Steuerfläche 78 gebildet ist.

Beim Verschieben des Kopplungselementes 36 wird auch der Ventilkörper 24 so in die Schließstellung verschoben, dass die Ventilöffnung 20 dichtend verschlossen wird. Die Ventileinrichtung 22 ist dadurch insgesamt verschlossen.

Der zuletzt erwähnte Abkühlvorgang ist in der Zeichnung nicht dargestellt. Am Ende dieses Vorgangs weist die Umlaufsperrvorrichtung 30 jedoch einen Zustand auf, der vorstehend bereits unter Verweis auf Figur 2 beschrieben wurde.

Das Rastelement 38 nimmt dabei erneut die Ausraststellung ein. In die Ausraststellung wurde das Rastelement 38 ausgehend von der Einraststellung in der Rastaufnahme 52 dadurch überführt, dass das Betätigungselement 40 vom ersten Zustand in den zweiten Zustand und erneut in den ersten Zustand überführt wurde, wobei vom Überführen vom zweiten Zustand in den ersten Zustand auch in diesem Fall das vorspannende Stellelement 68 ergänzend wirksam wurde.

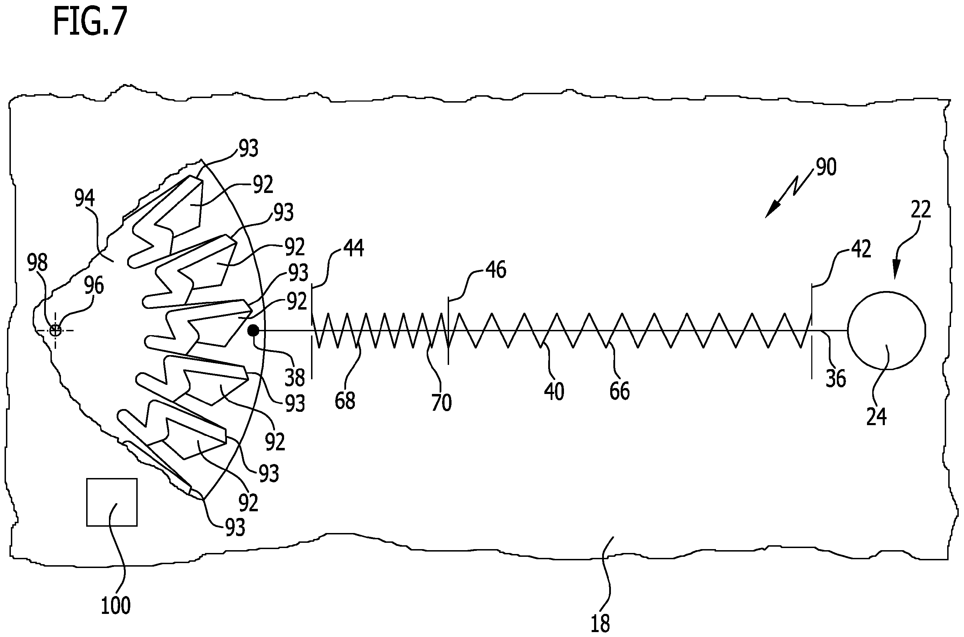

Figur 7 zeigt in schematischer Teildarstellung eine mit dem Bezugszeichen 90 belegte vorteilhafte Ausführungsform einer erfindungsgemäßen Umlaufsperrvorrichtung. Gleiche oder gleichwirkende Merkmale und Bauteile der Umlaufsperrvorrichtung 30 und 90 sind mit identischen Bezugszeichen belegt. Es wird nachfolgend nur auf die wesentlichen Unterschiede eingegangen. Auch die Umlaufsperrvorrichtung 90 könnte bei dem Sterilisierbehälter 10 anstelle der Umlaufsperrvorrichtung 30 zum Einsatz kommen.

Bei der Umlaufsperrvorrichtung 90 sind anstelle des nur einen Umlaufkörpers 32 eine Mehrzahl von identischen Umlaufkörpern 92 vorhanden. In funktioneller Hinsicht sind die Umlaufkörper 32 und 92 weitgehend identisch ausgestaltet. Allerdings weisen die Umlaufkörper 92 nicht das Lagerelement zur verschwenkbaren Lagerung an der Behälterwand 18 auf.

Außerdem ist anstelle des Anschlagglieds 86 ein Anschlagglied 93 vorgesehen, welches eine Neigung aufweist, die derjenigen der Steuerfläche 78 entgegengesetzt ist. Dies erlaubt es, das Rastelement 38 nach Durchlaufen von zwei Erwärmungs- und Abkühlungszyklen vor dem darauffolgenden Zyklus zum Eingang 72 des benachbarten Umlaufkörpers 92 zu führen.

Die Mehrzahl von Umlaufkörpern 92 sind an einem gemeinsamen Halteteil 94 festgelegt oder gebildet, das ein Lagerelement 96 umfasst. Das Lagerelement 96 definiert eine Drehachse 98, um das das Halteteil 94 an der Behälterwand 18 drehbar ist. Die Umlaufkörper 92 sind radial bezüglich der Drehachse am Halteteil 94 ausgerichtet und liegen nebeneinander.

Dabei sind die Umlaufkörper 92 so positioniert, dass einem jeweiligen Eingang 72 einer Zwangsführung 50 ein jeweiliger Ausgang 74 einer Zwangsführung eines benachbarten Umlaufkörpers 92 benachbart ist. Das Rastelement 38 kann nach Durchlaufen von zwei Erwärmungs- und Abkühlungszyklen mit Durchgang durch eine jeweilige Zwangsführung 50 eines Umlaufkörpers 92 beim darauffolgenden Erwärmungs- und Abkühlungszyklus in die Zwangsführung 50 eines benachbarten Umlaufkörpers 92 eintreten.

Mit dem Halteteil 94 ist ein Zählwerk 100 gekoppelt. Das Zählwerk 100 kann mechanisch und/oder elektrisch ausgestaltet sein und beispielsweise eine Anzeige umfassen, mit dem die Anzahl der Umdrehungen des Halteteils 94 gezählt werden können. Bei Vorhandensein von n Umlaufkörpern 92 an einem Halteteil 94 bedarf es 2n Erwärmungs- und Abkühlungszyklen, um eine Umdrehung des Halteteils 94 hervorzurufen. Dadurch lässt sich auch die Anzahl der Öffnungs- und Schließvorgänge der Ventileinrichtung 22 zählen.

### Bezugszeichenliste:

- 10: Sterilisierbehälter
- 12: Behälterinnenraum
- 14: Sterilisierbehälterwanne
- 16: Sterilisierbehälterdeckel
- 18: Behälterwand
- 20: Ventilöffnung
- 22: Ventileinrichtung
- 24: Ventilkörper
- 30: Umlaufsperrvorrichtung
- 32: Umlaufkörper
- 34: Wirkungselement
- 36: Kopplungselement
- 38: Rastelement
- 40: Betätigungselement
- 42: Anschlagelement
- 44: Anschlagelement
- 46: Anschlagelement
- 48: Lagerelement
- 50: Zwangsführung
- 52: Rastaufnahme
- 54: Eingangskanal
- 56: Rampe
- 58: Anschlagglied
- 60: Steuerfläche
- 62: Steuerfläche
- 64: Ausgangskanal
- 66: Federelement
- 68: Stellelement
- 70: Federelement
- 72: Eingang
- 74: Ausgang
- 76: Vorsprung
- 78: Steuerfläche
- 80: Drehachse
- 82: Ausnehmung
- 84: Rampe
- 86: Anschlagglied
- 90: Umlaufsperrvorrichtung
- 92: Umlaufkörper
- 93: Anschlagglied
- 94: Halteteil
- 96: Lagerelement
- 98: Drehachse
- 100: Zählwerk

## Patentansprüche

1. Medizinische Umlaufsperrvorrichtung, umfassend mindestens einen Umlaufkörper (32; 92) und ein Rastelement (38), wobei der mindestens eine Umlaufkörper (32; 92) eine Zwangsführung (50) und mindestens eine Rastaufnahme (52) für das Rastelement (38) aufweist, und wobei das Rastelement (38) durch die Zwangsführung (50) von einer Ausraststellung in eine Einraststellung an einer ersten Seite der mindestens einen Rastaufnahme (52) in dieselbe und an einer zweiten Seite der mindestens einen Rastaufnahme (52) von der Einraststellung wieder in eine Ausraststellung führbar ist, wobei die Umlaufsperrvorrichtung (30; 90) ein mit dem Rastelement (38) über ein Kopplungselement (36) gekoppeltes Wirkungselement (34) und ein mit dem Rastelement (38) in Wirkverbindung stehendes thermisches Betätigungselement (40) umfasst, das temperaturbedingt zum Bewegen des Wirkungselementes (34) von einem ersten Zustand in einen zweiten Zustand und umgekehrt überführbar ist, wobei der erste Zustand und der zweite Zustand einen Niedertemperaturzustand und einen Hochtemperaturzustand umfassen sowie einen Zustand geringerer Erstreckung und einen Zustand größerer Erstreckung, und wobei zum Überführen des Rastelementes (38) von der Ausraststellung in die Einraststellung in der mindestens einen Rastaufnahme (52) sowie zum Überführen des Rastelementes (38) von der Einraststellung in eine Ausraststellung das Betätigungselement (40) jeweils vom ersten Zustand in den zweiten Zustand und wieder in den ersten Zustand überführbar und dadurch das Rastelement (38) in der Zwangsführung bewegbar ist.

2. Umlaufsperrvorrichtung nach einem der voranstehenden Ansprüche, **dadurch gekennzeichnet, dass** das Betätigungselement (40) aus einem Phasenumwandlungsmaterial besteht oder ein solches umfasst, das im ersten Zustand und im zweiten Zustand unterschiedliche Phasenzustände einnimmt.

3. Umlaufsperrvorrichtung nach Anspruch 1 oder 2, **dadurch gekennzeichnet, dass** das Betätigungselement (40) aus einem Formgedächtnismaterial besteht oder ein solches umfasst.

4. Umlaufsperrvorrichtung nach Anspruch 3, **dadurch gekennzeichnet, dass** das Formgedächtnismaterial Nitinol ist.

5. Umlaufsperrvorrichtung nach einem der voranstehenden Ansprüche, **dadurch gekennzeichnet, dass** das Betätigungselement (40) schraubenförmig ist und das Kopplungselement (36) und/oder das Wirkungselement (34) umgibt.

6. Umlaufsperrvorrichtung nach einem der voranstehenden Ansprüche, **dadurch gekennzeichnet, dass** die Umlaufsperrvorrichtung (30; 90) ein Stellelement (68) umfasst, das mit dem Rastelement (38) in Wirkverbindung steht und dieses bewegt, wenn das Betätigungselement (40) vom Zustand größerer Erstreckung in den Zustand geringerer Erstreckung überführt wird, wobei das Stellelement (68) als Federelement (70) ausgestaltet ist.

7. Umlaufsperrvorrichtung nach Anspruch 6, **dadurch gekennzeichnet, dass** die Umlaufsperrvorrichtung (30; 90) mindestens ein am Kopplungselement (36) festgelegtes Anschlagelement (46) für das Betätigungselement (40) und/oder für das Stellelement (68) aufweist oder bildet.

8. Umlaufsperrvorrichtung nach einem der voranstehenden Ansprüche, **dadurch gekennzeichnet, dass** das Wirkungselement (34) ein(en) Ventilkörper (24) einer Ventileinrichtung (22) ist oder umfasst.

9. Umlaufsperrvorrichtung nach einem der voranstehenden Ansprüche, **dadurch gekennzeichnet, dass** das Kopplungselement (36) einstückig mit dem Rastelement (38) und/oder mit dem Wirkungselement (34) gebildet ist.

10. Umlaufsperrvorrichtung nach einem der voranstehenden Ansprüche, **dadurch gekennzeichnet, dass** die Zwangsführung (50) eine Kulissenführung ist.

11. Umlaufsperrvorrichtung nach einem der voranstehenden Ansprüche, **dadurch gekennzeichnet, dass** die mindestens eine Rastaufnahme (52) als Rastrücksprung am mindestens einen Umlaufkörper (32; 92) ausgebildet ist.

12. Umlaufsperrvorrichtung nach einem der voranstehenden Ansprüche, **dadurch gekennzeichnet, dass** die Zwangsführung (50) eine Rampe (56) für das Rastelement (38) aufweist, über welche dieses beim Überführen von der Ausraststellung in die Einraststellung gleitet, wobei die Zwangsführung (50) ein das Rastelement (38) nach dem Übergleiten zurückhaltendes Anschlagglied (58) umfasst, an dem eine das Rastelement (38) in die Rastaufnahme (52) führende Steuerfläche (60) angeordnet ist, wobei die Zwangsführung (50) einen die Rampe (56) bildenden Eingangskanal (54) für das Rastelement (38) aufweist.

13. Umlaufsperrvorrichtung nach einem der voranstehenden Ansprüche, **dadurch gekennzeichnet, dass** der mindestens eine Umlaufkörper (32; 92) genau eine Rastaufnahme (52) aufweist, ausgehend von der das Rastelement (38) in die Ausraststellung überführbar ist.

14. Umlaufsperrvorrichtung nach einem der Ansprüche 1 bis 12, **dadurch gekennzeichnet, dass** der mindestens eine Umlaufkörper mindestens eine weitere Rastaufnahme für das Rastelement aufweist, in der dieses eine weitere Einraststellung einnehmen kann, wobei zum Überführen des Rastelementes von der Einraststellung in die weitere Einraststellung das Betätigungselement vom zweiten Zustand in den ersten Zustand und wieder in den zweiten Zustand überführbar ist und dadurch das Rastelement in der Zwangsführung bewegbar ist.

15. Umlaufsperrvorrichtung nach einem der voranstehenden Ansprüche, **dadurch gekennzeichnet, dass** die Umlaufsperrvorrichtung (90) eine Mehrzahl von an einem gemeinsamen Halteteil (94) festgelegten oder gebildeten Umlaufkörpern (92) aufweist, wobei ein jeweiliger Ausgang (74) einer Zwangsführung (50) eines Umlaufkörpers (92) in einen jeweiligen Eingang (72) einer Zwangsführung (50) eines benachbarten Umlaufkörpers (92) mündet oder diesem benachbart ist.

## Claims

1. Medical circulation detent device, comprising at least one circulation body (32; 92) and a latching element (38), the at least one circulation body (32; 92) comprising a restricted guide (50) and at least one latching receptacle (52) for the latching element (38), and the latching element (38) being guidable through the restricted guide (50) from an unlatched position to a latched position at a first side of the at least one latching receptacle (52) into the latter, and at a second side of the at least one latching receptacle (52) from the latched position to an unlatched position again, wherein the circulation detent device (30; 90) comprises an operative element (34) coupled to the latching element (38) via a coupling element (36), and a thermal actuating element (40) operatively connected to the latching element (38) and transferable due to temperature from a first state to a second state and vice versa in order to move the operative element (34), the first state and the second state comprising a low-temperature state and a high-temperature state and also a state of lesser extent and a state of greater extent, and wherein in order to transfer the latching element (38) from the unlatched position to the latched position in the at least one latching receptacle (52) and in order to transfer the latching element (38) from the latched position to an unlatched position, the actuating element (40) is respectively transferable from the first state to the second state and to the first state again, and the latching element (38) is thereby movable in the restricted guide.

2. Circulation detent device in accordance with any one of the preceding claims, **characterized in that** the actuating element (40) consists of or comprises a phase transformation material, which assumes different phase states in the first state and the second state.

3. Circulation detent device in accordance with claim 1 or 2, **characterized in that** the actuating element (40) consists of or comprises a shape memory material.

4. Circulation detent device in accordance with claim 3, **characterized in that** the shape memory material is nitinol.

5. Circulation detent device in accordance with any one of the preceding claims, **characterized in that** the actuating element (40) is helical and surrounds the coupling element (36) and/or the operative element (34).

6. Circulation detent device in accordance with any one of the preceding claims, **characterized in that** the circulation detent device (30; 90) comprises a setting element (68) which is operatively connected to the latching element (38) and moves it when the actuating element (40) is transferred from the state of greater extent to the state of lesser extent, wherein the setting element (68) is configured as spring element (70).

7. Circulation detent device in accordance with claim 6, **characterized in that** the circulation detent device (30; 90) comprises or forms at least one stop element (46), which is fixed to the coupling element (36), for the actuating element (40) and/or for the setting element (68).

8. Circulation detent device in accordance with any one of the preceding claims, **characterized in that** the operative element (34) is or comprises a valve body (24) of a valve device (20).

9. Circulation detent device in accordance with any one of the preceding claims, **characterized in that** the coupling element (36) is formed in one piece with the latching element (38) and/or with the operative element (34).

10. Circulation detent device in accordance with any one of the preceding claims, **characterized in that** the restricted guide (50) is a link guide.

11. Circulation detent device in accordance with any one of the preceding claims, **characterized in that** the at least one latching receptacle (52) is formed as latching recess on the at least one circulation body (32; 92).

12. Circulation detent device in accordance with any one of the preceding claims, **characterized in that** the restricted guide (50) comprises a ramp (56) for the latching element (38), over which the latter slides during transfer from the unlatched position to the latched position, and the restricted guide (50) comprises a stop member (58) which holds the latching element (38) back after the sliding-over, and on which a control surface (60) guiding the latching element (38) into the latching receptacle (52) is arranged, wherein the restricted guide (50) comprises an entrance channel (54), forming the ramp (56), for the latching element (38).

13. Circulation detent device in accordance with any one of the preceding claims, **characterized in that** the at least one circulation body (32; 92) comprises precisely one latching receptacle (52), starting from which the latching element (38) is transferable to the unlatched position.

14. Circulation detent device in accordance with any one of claims 1 to 12, **characterized in that** the at least one circulation body comprises at least one further latching receptacle for the latching element, in which the latter can assume a further latched position, the actuating element being transferable from the second state to the first state and to the second state again, and the latching element thereby being movable in the restricted guide, in order to transfer the latching element from the latched position to the further latched position.

15. Circulation detent device in accordance with any one of the preceding claims, **characterized in that** the circulation detent device (90) comprises a plurality of circulation bodies (92) fixed or formed on a common holding part (94), a respective exit (74) of a restricted guide (50) of a circulation body (92) opening into or being adjacent to a respective entrance (72) of a restricted guide (50) of an adjacent circulation body (92).

## Revendications

1. Dispositif de blocage en rotation destiné à un usage dans le domaine médical, comprenant au moins un corps rotatif (32; 92) et un élément d'encliquetage (38), dispositif
dans lequel ledit au moins un corps rotatif (32; 92) présente un guidage forcé (50) et au moins un logement d'encliquetage (52) pour l'élément d'encliquetage (38), et
dans lequel l'élément d'encliquetage (38) peut être transféré par le guidage forcé (50), d'une position de désencliquetage à une position d'encliquetage sur un premier côté dudit au moins un logement d'encliquetage (52), dans celui-ci, et sur un deuxième côté dudit au moins un logement d'encliquetage (52), de la position d'encliquetage à nouveau dans une position de désencliquetage,
le dispositif de blocage en rotation (30; 90) comprenant un élément d'action (34) couplé à l'élément d'encliquetage (38) par l'intermédiaire d'un élément de couplage (36), et un élément d'actionnement thermique (40), qui est en liaison d'interaction avec l'élément d'encliquetage (38), et qui, en vue de déplacer l'élément d'action (34), peut être transféré en fonction de la température, d'un premier état à un deuxième état et inversement, dispositif
dans lequel le premier état et le deuxième état comprennent un état de basse température et un état de haute température, ainsi qu'un état d'étendue moindre et un état de plus grande étendue, et
dans lequel pour transférer l'élément d'encliquetage (38) de la position de désencliquetage à la position d'encliquetage dans ledit au moins un logement d'encliquetage (52), ainsi que pour transférer l'élément d'encliquetage (38) de la position d'encliquetage dans une position de désencliquetage, l'élément d'actionnement (40) peut être transféré respectivement du premier état au deuxième état et à nouveau dans le premier état, en permettant ainsi le déplacement de l'élément d'encliquetage (38) dans le guidage forcé.

2. Dispositif de blocage en rotation selon l'une des revendications précédentes, **caractérisé en ce que** l'élément d'actionnement (40) est réalisé en un matériau à changement de phase ou comprend un tel matériau, qui prend des états de phase différents dans le premier état et dans le second état.

3. Dispositif de blocage en rotation selon la revendication 1 ou la revendication 2, **caractérisé en ce que** l'élément d'actionnement (40) est réalisé en un matériau à mémoire de forme ou comprend un tel matériau.

4. Dispositif de blocage en rotation selon la revendication 3, **caractérisé en ce que** le matériau à mémoire de forme est du Nitinol.

5. Dispositif de blocage en rotation selon l'une des revendications précédentes, **caractérisé en ce que** l'élément d'actionnement (40) est de forme hélicoïdale et entoure l'élément de couplage (36) et/ou l'élément d'action (34).

6. Dispositif de blocage en rotation selon l'une des revendications précédentes, **caractérisé en ce que** le dispositif de blocage en rotation (30; 90) comprend un élément actionneur (68), qui est en liaison d'interaction avec l'élément d'encliquetage (38) et déplace celui-ci lorsque l'élément d'actionnement (40) est transféré de l'état de plus grande étendue à l'état d'étendue moindre, l'élément de déplacement (68) étant réalisé sous la forme d'un élément de ressort (70).

7. Dispositif de blocage en rotation selon la revendication 6, **caractérisé en ce que** le dispositif de blocage en rotation (30; 90) présente ou forme au moins un élément de butée (46), fixé à l'élément de couplage (36), pour l'élément d'actionnement (40) et/ou à l'élément de déplacement (68).

8. Dispositif de blocage en rotation selon l'une des revendications précédentes, **caractérisé en ce que** l'élément d'action (34) est ou comprend un corps d'obturation de soupape (24) d'un dispositif de soupape (22) .

9. Dispositif de blocage en rotation selon l'une des revendications précédentes, **caractérisé en ce que** l'élément de couplage (36) est formé d'un seul tenant avec l'élément d'encliquetage (38) et/ou avec l'élément d'action (34).

10. Dispositif de blocage en rotation selon l'une des revendications précédentes, **caractérisé en ce que** le guidage forcé (50) est un guidage à coulisse.

11. Dispositif de blocage en rotation selon l'une des revendications précédentes, **caractérisé en ce que** ledit au moins un logement d'encliquetage (52) est réalisé en tant que décrochement d'encliquetage en retrait sur ledit au moins un corps rotatif (32; 92).

12. Dispositif de blocage en rotation selon l'une des revendications précédentes, **caractérisé en ce que** le guidage forcé (50) présente une rampe (56) pour l'élément d'encliquetage (38), par-dessus laquelle glisse ce dernier lors du transfert de la position de désencliquetage à la position d'encliquetage, le guidage forcé (50) comportant un organe de butée (58) qui retient l'élément d'encliquetage (38) après son passage glissant, et sur laquelle est agencée une surface de commande (60) guidant l'élément d'encliquetage (38) dans le logement d'encliquetage (52), le guidage forcé (50) présentant un canal d'entrée (54) formant la rampe (56), pour l'élément d'encliquetage (38).

13. Dispositif de blocage en rotation selon l'une des revendications précédentes, **caractérisé en ce que** ledit au moins un corps rotatif (32; 92) présente exactement un logement d'encliquetage (52) à partir duquel l'élément d'encliquetage (38) peut être transféré dans la position de désencliquetage.

14. Dispositif de blocage en rotation selon l'une des revendications 1 à 12, **caractérisé en ce que** ledit au moins un corps rotatif présente au moins un autre logement d'encliquetage pour l'élément d'encliquetage, dans lequel ce dernier peut prendre une autre position d'encliquetage, le transfert de l'élément d'encliquetage de la position d'encliquetage à ladite autre position d'encliquetage étant obtenu grâce au fait que l'élément d'actionnement peut être transféré du deuxième état au premier état et à nouveau au deuxième état, en permettant ainsi le déplacement de l'élément d'encliquetage dans le guidage forcé.

15. Dispositif de blocage en rotation selon l'une des revendications précédentes, **caractérisé en ce que** le dispositif de blocage en rotation (90) comporte une pluralité de corps rotatifs (92) fixés ou formés sur une pièce de maintien (94) commune, une sortie (74) respective d'un guidage forcé (50) d'un corps rotatif (92) débouchant dans une entrée (72) respective d'un guidage forcé (50) d'un corps rotatif (92) voisin, ou bien étant voisine de celui-ci.
